# EUROPEAN PATENT APPLICATION

(11) **EP 0 610 551 A1**
(43) Date of publication of application: **17.08.1994**
(21) Application number: 93116978.3
(22) Date of filing: 20.10.1993
(51) Int. Cl.: C07C 247/04

(54) **Improved method of azidohydrocarbon production**

(30) Priority: 08.02.1993 US 14948
(71) Applicant: ROCKWELL INTERNATIONAL CORPORATION, Seal Beach, California 90740-8250 (US)
(72) Inventor: Bauerle, George Louis, Simi Valley, California 93063 (US); Weber, James Frederick, Moorpark, California 93021 (US); Flanagan, Joseph Edward, Woodland Hill, California 91367 (US)
(74) Representative: Wächtershäuser, Günter, Prof. Dr.

(57) **Abstract**

Disclosed is a process for producing an azidohydrocarbon comprising combining in kerosene, sodium azide, a catalytic amount of a phase transfer catalyst and a halogenated hydrocarbon, initiating the reaction and recovering the reaction product azidohydrocarbon.

## Description

### BACKGROUND OF THE INVENTION

This invention relates generally to an energetic composition, a process for its preparation and its uses. More particularly it relates to azidohydrocarbons such as 1,6-diazidohexane, an energetic diazidoalkane useful as a component in a propellant or combustion system.

### SUMMARY OF THE INVENTION

Accordingly, it is an object of this invention to provide energetic azidohydrocarbons which are relatively insensitive to impact and are thermally stable.

Another object of this invention is to provide a process of preparation of the diazidoalkane 1,6-diazidohexane with the aforementioned properties.

According to the invention, these and other objects are attained by providing a process for producing azidohydrocarbons comprising introducing at ambient temperature and pressure into a suitable reaction vessel kerosene as a reactant carrier. To the kerosene is admixed sodium azide and a phase transfer catalyst followed by the addition of halogenated hydrocarbons under controlled conditions, reacting the mixture and recovering the reaction product azidohydrocarbon.

The preferred class Of phase transfer catalysts comprises tetraalkylammonium and tetraalkylphosphonium salts which include tetrabutylammonium chloride, tetrabutylammonium bromide, tetrabutylammonium azide, tetrabutylammonium hydrogen sulfate, methyltributylammonium chloride, methyltributylammonium azide, tricaprylmethylammonium chloride, tetraethylammonium azide, tetraethylammonium chloride, tetrabutylphosphonium chloride, tetrabutylphosphonium bromide, tetrabutylphosphonium azide, tetrapropylphosphonium chloride, tetrapropylphosphonium bromide, and tetrapropylphosphonium azide.

### DESCRIPTION OF THE PREFERRED ENBODIMENT

Although the azidohydrocarbon of the present invention may be prepared as described above, there are some recommended steps to be followed if a product of high purity and yield is to be realized. The product and process of the invention are further described and illustrated by the example hereinunder set forth.

### EXAMPLE

In a suitable reaction vessel equipped with a stirrer and heater element was introduced at ambient temperature and pressure about 100 grams of kerosene of C₁₂ to C₁₈. To the kerosene was next added sodium azide (NaN₃) (71 gm. 1.09 mole or 2.4% excess) and 31 grams (0.11 mole) of tetrabutylammonium azide [CH₃(CH₂)₃]₄N-N₃ to form a slurry. Next, 81 grams (0.523 mole) of 1,6 dichlorohexane [Cl(CH₂)₆Cl] was added to the slurry mixture over a period of about 25 minutes. The reaction was initiated at about 100°C for about 45 minutes and then at 105°C for about 2 hours and 15 minutes. The resulting crude product (78 grams/89%) 1,6-diazidohexane [N₃(CH₂)₆N₃] was water washed and then dried over silica and alumina. The three hour total reaction time is superior to known alkane azidation procedures for preparing 1,6-diazidohexane.

As will be apparent to those skilled in the art, various modifications of the invention can be made or followed in view of the above disclosure without departing from the spirit and scope of the invention.

## Claims

1. A process for producing an azidohydrocarbon comprising introducing at ambient temperature and pressure into a suitable reaction vessel, kerosene; admixing with the kerosene sodium azide and a catalytic amount of a phase transfer catalyst followed by the addition of a halogenated hydrocarbon into the mixture; initiating a reaction of the sodium azide, catalyst and halogenated hydrocarbon; and recovering the reaction product azidohydrocarbon.

2. The process of claim 1 wherein the initiation step starts at about 100°C for about 45 minutes and then at about 105°C for about 2 hours and 15 minutes.

3. The process of claim 1 wherein the halogenated hydrocarbon was introduced into the reaction vessel over a period of about 25 minutes.

4. The process of claim 1 wherein the azidohydrocarbon was removed from the reaction vessel, water washed and then dried over silica and alumina.

5. A process for producing an azidohydrocarbon according to claim 1 wherein the phase transfer catalyst is a tetraalkylammonium salt.

6. A process for producing an azidohydrocarbon according to claim 1 wherein the phase transfer catalyst is a tetraalkylphosphonium salt.

7. A process for producing an azidohydrocarbon according to claim 1 wherein the phase transfer catalyst is tetrabutylammonium chloride, tetrabutylammonium bromide, tetrabutylammonium azide, tetrabutylammonium hydrogen sulfate, methyltributylammonium chloride, methyltributylammonium azide, tricaprylmethylammonium chloride, tetraethylammonium azide, tetraethylammonium chloride, tetrabutylphosphonium chloride, tetrabutylphosphonium bromide, tetrabutylphosphonium azide, tetrapropylphosphonium chloride, tetrapropylphosphonium bromide, or tetrapropylphosphonium azide.

8. A process for producing 1,6-diazidohexane comprising introducing at ambient temperature and pressure into a suitable reaction vessel, kerosene; admixing with the kerosene sodium azide and a catalytic amount of tetrabutylammonium azide followed by the addition of 1,6-dichlorohexane into the mixture; initiating a reaction of the sodium azide, tetrabutylammonium azide and 1,6-dichlorohexane; and recovering the reaction product 1,6-diazidohexane.
